Europäisches Patentamt

European Patent Office

Office européen des brevets

(11) Veröffentlichungsnummer: **0 136 502**

**A2**

# EUROPÄISCHE PATENTANMELDUNG

(21) Anmeldenummer: 84109805.6

(22) Anmeldetag: 17.08.84

(51) Int. Cl.⁴: **A 61 K 31/725**

---

(30) Priorität: 07.09.83 DE 3332301

(43) Veröffentlichungstag der Anmeldung:
10.04.85 Patentblatt 85/15

(84) Benannte Vertragsstaaten:
AT BE CH DE FR GB IT LI NL SE

(71) Anmelder: Algina Aktiengesellschaft
c/o Rensch Treuhand Baarer Strasse 43
CH-6301 Zug(CH)

(72) Erfinder: Kulbe, Klaus Dieter, Dr.
Ritterstrasse 12
D-7031 Gärtringen 2(DE)

(72) Erfinder: Weber, Hans, Dr.
Bittenfelder Strasse 8
D-7140 Ludwigsburg(DE)

(74) Vertreter: Kraus, Walter, Dr. et al,
Patentanwälte Kraus, Weisert & Partner Irmgardstrasse
15
D-8000 München 71(DE)

---

(54) Verwendung von Eisen(II)-, Zink- und Magnesiumsalzen und ihren Mischsalzen zur Substitution von Eisen, Zink und Magnesium.

(57) Es wird die Verwendung von Eisen (II)-, Zink- und Magnesiumsalzen und ihren Mischsalzen, der Alginsäure, der Pektinsäure, einer natürlichen polymeren, anionischen Carbonsäure aus der Gruppe Polymannuronsäure, Polygalacturonsäure, Polyglucuronsäure, Polyguluronsäure und Polyiduronsäure oder ihren Gemischen zur Substitution von Eisen, Zink und Magnesium beschrieben.

0136502

AW/fe

ALGINA AKTIENGESELLSCHAFT
CH-6301 Zug

Verwendung von Eisen(II)-, Zink- und Magnesiumsalzen und ihren Mischsalzen zur Substitution von Eisen, Zink und Magnesium

## B E S C H R E I B U N G

Die Erfindung betrifft die Verwendung von Eisen(II)-, Zink- und Magnesiumsalzen und ihren Mischsalzen zur Substitution von Eisen, Zink und/oder Magnesium.

Mineralstoffe mit Spurenelementcharakter, wie beispielsweise Eisen, Zink und Magnesium, sind für den Menschen unentbehrlich. Die Mineralstoffe werden mit den Nährstoffen in den Organismus aufgenommen und zu einem großen Teil durch die natürlichen Ausscheidungen, wie Kot, Harn und Schweiß, wieder abgegeben und müssen daher ergänzt werden, damit ein ausgeglichener Mineralstoffkreislauf gewährleistet ist.

Oft treten jedoch latente oder akute Mineralstoffmangelerscheinungen auf, oder es besteht, wie beispielsweise bei der Schwangerschaft, Stillzeit und Zahnbildung, ein erhöhter Bedarf an Mineralstoffen. Bei der Prophylaxe und Therapie der durch Streß bedingten Herzerkrankungen spielt

- 2 -    0136502

der Magnesiummangel eine große Rolle.

Die Eisenmangelanämie ist auch heute noch eine Krankheit, die nur schwer zu behandeln ist. Um dem Organismus Eisen zuzuführen, muß dieses vom Körper resorbiert werden. Metallisches Eisen wird im Darm nicht aufgenommen, und man verwendet daher heute Präparate, die zweiwertige Eisensalze enthalten. Diese Eisenpräparate werden jedoch ebenfalls nicht vollständig resorbiert, und die richtige Dosierung der Eisenzufuhr ist extrem schwierig. Wird zuviel Eisen auf einmal zugeführt, können sich Übelkeit, Erbrechen und Magenschmerzen einstellen. Besonders bei Kindern ist die richtige Dosierung des Eisens sehr schwer zu erreichen.

Die heute verwendeten Stoffe zur Einstellung des Mineralstoffwechsels sind in vielen Fällen einfache Metallsalze, wie die Chloride, Sulfate und Carbonate, die in weitgehend dissoziierter Form vorliegen und damit zu einer Reihe von Nebenwirkungen Anlaß geben. Einige Präparate wurden mit entsprechenden Zusätzen versehen. Die Zusätze bewirkten jedoch nur, daß der Geschmack verbessert werden konnte, die Nebenwirkungen jedoch weitgehend unverändert blieben. Im allgemeinen werden in mehr oder weniger starkem Ausmaß folgende Nebenwirkungen beobachtet: Magenreizung, unangenehmer Geschmack im Mund, Übelkeit, Erbrechen und Durchfall.

Der vorliegenden Erfindung liegt die Aufgabe zugrunde, Stoffe zur Verfügung zu stellen, die zur Erhöhung der Magnesium-, Eisen- und Zinkspiegel verwendet werden können und welche die Nachteile der im Handel befindlichen Präparate nicht aufweisen.

Überraschenderweise wurde gefunden, daß Metallsalze und Metallmischsalze, die Kationenaustauscher auf der Basis von natürlich vorkommenden Polymeren oder deren chemischen Derivaten darstellen, als Arzneimittel Verwendung finden können, insbesondere als Mittel zur Substitution von Magnesium, Zink und Eisen.

Gegenstand der Erfindung ist die Verwendung von Eisen(II)-, Zink- und Magnesiumsalzen und ihren Mischsalzen

(1) der Alginsäure,

(2) der Pektinsäure,

(3) einer natürlichen polymeren, anionischen Carbonsäure aus der Gruppe Polymannuronsäure, Polygalacturonsäure, Polyglucuronsäure, Polyguluronsäure und Polyiduronsäure und ihren Gemischen

zur Substitution von Eisen, Zink und Magnesium.

Wenn in der vorliegenden Anmeldung von Salzen oder Mischsalzen gesprochen wird, werden hierunter Verbindungen verstanden, die in stöchiometrischer oder nichtstöchiometrischer Form vorliegen können. Beispielsweise kann das Metall in den Salzen und Mischsalzen in einer Menge im Bereich der 0,5- bis 3fachen, bevorzugt der 0,5- bis 2fachen und besonders bevorzugt der 0,8- bis 1,5fachen, Menge der stöchiometrisch erforderlichen Menge vorhanden sein. Ganz besonders bevorzugte Verbindungen sind solche, in denen das Metall in praktisch der stöchiometrischen Menge vorhanden ist.

Bevorzugt werden erfindungsgemäß Eisen(II)-alginat, Magnesiumalginat und Zinkalginat als Salze verwendet.
Beispiele für Mischsalze sind Eisen(II)-, Zinkalginat,
Magnesiumalginat und die entsprechenden
Pektinate.

Die Eisen(II)-, Zink- und Magnesiumsalze werden in an
sich bekannter Weise hergestellt. Beispielsweise gibt man
zu einer 2- bis 20%igen Lösung der entsprechenden Säure ein
Alkalihydroxid bis zu einem pH-Wert von 5,0 bis 8,0. Die
Lösung wird dann mit einer 0,2 bis 2 M Lösung eines
physiologisch annehmbaren Eisen(II)-, Zink- oder Magnesiumsalzes versetzt, wobei die Zugabe der Salzlösung
bevorzugt tropfenweise erfolgt. Ein solches Salzgemisch kann
anschließend 1 bis 20 Stunden bei einer Temperatur von
2 bis 50°C stehengelassen werden. Das Produkt wird abfiltriert, gewaschen und getrocknet.

Die Mischsalze kann man beispielsweise herstellen, indem
man zu einer 2-bis 20%igen wäßrigen Lösung der Säure ein
Alkalihydroxid bis zu einem pH-Wert von 5,0 bis 8,0 hinzugibt, die Lösung bei einer Temperatur im Bereich von
0 bis 50°C in eine 0,2 bis 2 M Lösung eines physiologisch
annehmbaren Eisen(II)-salzes gibt und zu dieser Lösung
dann die Lösung eines Zink- oder Magnesiumsalzes zutropft.
Das Reaktionsgemisch wird dann 1 bis 20 Stunden bei einer
Temperatur von 2 bis 50°C stehengelassen, das Reaktionsprodukt wird abfiltriert, der Niederschlag bis zum erforderlichen Restgehalt an freien Ionen gewaschen und
schließlich getrocknet.

Überraschenderweise wurde jetzt gefunden, daß die oben
erwähnten Salze und Mischsalze zur schonenden und langwirkenden Substitution bestimmter Metallionen, wie $Fe^{2+}$,
$Mg^{2+}$ und $Zn^{2+}$, bei entsprechenden prälatenten, latenten
oder akuten bzw. manifesten Mineralstoffmangelerscheinungen oder erhöhtem Bedarf an Mineralstoffen, wie zum

- 5 -                    0136502

Beispiel bei der Schwangerschaft, in der Stillzeit und während der Zahnbildung (Fe-Antianämika), sowie bei der Prophylaxe und Therapie der durch Mg-Mangel und Streß bedingten Herzerkrankungen  verwendet werden können.

### Vorteile der neuen Präparate

Durch Bindung der Metallionen an natürliche polymere Anionen entstehen Kationenaustauscher, die aufgrund ihrer Kohlenhydratnatur sehr gut vertragen werden, ihre Kationen langsam freisetzen bzw. austauschen (Retardwirkung) und damit unerwünschte Nebenwirkungen vermeiden, wie sie in mehr oder weniger großem Umfang bei allen üblichen Mineralpräparaten beobachtet werden, bei denen durch schnelle Freisetzung bereits im Magen hohe Ionenkonzentrationen auftreten, die eine Reizung der Schleimhäute zur Folge haben können. Die wasserunlöslichen Salze, wie das Eisen(II)-alginat und Zinkalginat, quellen dagegen im Magen-Darm-Bereich zunächst auf, lösen sich insbesondere in Gegenwart von zugesetztem oder Nahrungsphosphat allmählich auf und entfalten erst damit ihre eigentliche Ionenaustauscherwirkung. Magnesiumpräparate sind wasserlöslich und kommen damit zusätzlich für intravenöse Applikation in Betracht.

Gut gewaschene, von einem Überschuß an freien Kationen befreite erfindungsgemäß verwendete Salze oder Mischsalze zeichnen sich deshalb wegen ihrer guten Verträglichkeit aus. Die mit entsprechenden Präparationen auf zum Beispiel Chlorid-, Sulfat- und Carbonatbasis üblichen Nebenwirkungen, wie Metallgeschmack auf der Zunge, Verfärbung (bei Eisenpräparaten) der Zunge und der Zähne, Kopfschmerzen, Müdigkeit und Erbrechen, gastrointestinale Beschwerden, wie Sodbrennen, Übelkeit und Diarrhoe, lassen sich weitestgehend vermeiden.

Ein weiterer besonderer Vorteil der erfindungsgemäß verwendeten Salze oder Mischsalze ist ihre biologische Herkunft. So gelten Alginsäure und Pektinsäure lebensmittelrechtlich als unbedenklich und dürfen in unbegrenzter Menge eingesetzt werden. Dies ist besonders vorteilhaft, wenn die langjährige chronische Anwendung eines bestimmten Arzneimittels erforderlich ist [vgl. J. Schormüller, Lehrbuch der Lebensmittelchemie, 2. Auflage (1974), Seiten 305, 100 - 109, Springer-Verlag, Berlin, Heidelberg, New York; 3. Verordnung über die Zulassung von Zusatzstoffen zu Lebensmitteln (Z Zul V) vom 22. Dezember 1981].

Die verwendeten Substanzen basieren auf Polysaccharidstrukturen, deren Grundbausteine von Glucose, Mannose, Galactose und Gulose abgeleitet sind und damit den bisher zum Teil benutzten Formulierungen mit Zuckerzusätzen ähneln.

Alginsäurederivate sind auch dadurch besonders verträglich, daß sie eine Schutzschicht auf der Magenschleimhaut auszubilden vermögen.

Es ist ohne Schwierigkeiten möglich, eine Reihe weiterer Pharmaka, wie sie bei anderen Kombinationspräparaten üblich sind, mit in das jeweilige Präparat zu integrieren.

Erfindungsgemäß können als Ausgangsmaterialien für die Herstellung der Salze und Mischsalze verwendet werden:

1. Alginsäure
   Bevorzugt werden die in der Natur gefundenen Alginsäuren, die zwischen 0 und 100% Mannuronsäure und 0 bis 70% Guluronsäure enthalten, verwendet.

2. Pektinsäure

3. eine natürliche polymere anionische Carbonsäure aus der folgenden Gruppe:

a) Polymannuronsäure, wie zum Beispiel Alginsäure (Alginsäure wird erfindungsgemäß besonders bevorzugt),

b) Polygalacturonsäure, wie zum Beispiel Pektinsäure oder Pektine mit niedriger Methanolveresterung,

c) Polyglucuronsäure, beispielsweise solche, die durch mikrobielle Oxidation von Cellulose hergestellt wird,

d) Polyguluronsäure, beispielsweise bestimmte Alginsäuren, und

e) Polyiduronsäure oder ihre Gemische.

Selbstverständlich können auch Gemische der genannten Säuren 1) bis 3) eingesetzt werden.

Bevorzugt verwendet man Polysaccharide. Diese gehören zur Stoffklasse der Kohlenhydrate und sind hochmolekulare Verbindungen, die aus glykosidisch verknüpften Monosaccharideinheiten aufgebaut sind. Polysaccharide bilden mit Wasser hochviskose Lösungen oder in Gegenwart von Ionen Gele und finden als Verdickungs- und Geliermittel für Lebensmittel sowie in der Pharmazie als Träger- und Hüllsubstanzen für Medikamente Verwendung.

Zur Eisensubstitution werden Fe(II)-alginat und Eisen(II)-Magnesiumalginat bevorzugt und für die Nieren- und Harnsteinprophylaxe Magnesiumalginat.

- 8 - 0136502

Im folgenden werden einige erfindungsgemäße Anwendungsmöglichkeiten erläutert:

1. Fe-II-alginat

zur Substitution als gutverträgliches blutbildendes Mittel (Antianämikum)
- bei Eisenmangelzuständen, akuten und chronischen Blutungsanämien,
- zur Therapie und Prophylaxe von Eisenmangelzuständen, zum Beispiel bei erhöhtem Eisenbedarf während Schwangerschaft, Stillzeit, Wachstum,
- bei prälatentem und latentem Eisenmangel, Eisenmangelanämien; manifestem Eisenmangel,
- bei Regelblutungen,
- bei Eisenresorptionsstörungen,
- bei alimentärem Eisenmangel von Säuglingen, Kleinkindern und Jugendlichen; zur Eisenmangelprophylaxe bei Säuglingen und Kleinkindern,
- zur Prophylaxe des Eisenmangels bei Blutspendern,
- bei Eisenmangel nach akuten und chronischen Blutungen, Operationen; Rekonvaleszenz nach Blutverlusten,
- bei erhöhtem Bedarf während der Schwangerschaften, bei Wachstumsstörungen, bei eisenarmer Diät; Erschöpfungszuständen,
- bei Dauerdialysepatienten.

2. Magnesiumalginat

- zur Vermeidung alimentären Magnesiummangels (zum Beispiel bei Eiweißdiät),
- bei primärem und sekundärem Magnesiummangelsyndrom,
- besonders zur Prophylaxe und Therapie der durch Mg-Mangel und Streß bedingten Herzerkrankungen (zum Beispiel Prophylaxe von Myokardnekrosen und funktionell bedingten Herzrhythmusstörungen, vegetativer Übererregbarkeit),

- während Schwangerschaft und Stillzeit,
- bei Einnahme von Kontrazeptiva,
- bei Mg-Mangel von Leberkranken und Diabetikern,
- bei chronischem Alkoholmißbrauch,
- bei Thrombosegefährdung (Thrombo-Emboli-Prophylaxe),
- zur Zusatztherapie bei Pankreatitis, Leberzirrhose, Hypercholesterinämie, Arteriosklerose,
- als Adjuvans bei essentieller Hypertonie,
- zur Therapie von Myalgie, nächtlichen Wadenkrämpfen, Migräne u.a.,
- bei Calciumstoffwechselstörungen,
- bei der Verabreichung von Urolithiasismitteln auf der Basis von Na-Cellulosephosphat für Ca-haltige Nierensteine

3. <u>Zinkalginat</u>

Substitution bei
- entzündlichen Formen von Acne vulgaris
- Wundheilungsstörungen (Chirurgie),
- Acrodermatitis enterohepatica,
- Katabolie,
- zur ergänzenden Behandlung bei Diabetes mellitus,
- Prostatitis,
- geriatrischen Indikationen,
- virilen Potenzstörungen,
- als Abwehrschutz gegen langdauernde Infekte.

Die erfindungsgemäßen Salze und Mischsalze können in an sich bekannter Weise zu Kapseln, Tabletten, Granulat, Dragees formuliert werden. Die Dosierung hängt von der Art des verwendeten Salzes, dem Körpergewicht des Patienten und der erwünschten Substitution ab und kann vom Arzt auf einfache Weise bestimmt werden.

Die folgenden Beispiele erläutern die Erfindung.

B e i s p i e l   1

Darstellung von Eisen(II)-alginat

8 g Natriumalginat (Protanal LFR 5/60) werden in 100 ml
$H_2O$ gelöst, das zum Entgasen frisch ausgekocht wurde.
Die Natriumalginatlösung wird langsam unter leichtem
Rühren in eine mit NaOH auf pH 6 gebrachte 0,5 M $FeSO_4$-
Lösung getropft, die ebenfalls mit frisch ausgekochtem
Wasser bereitet wurde. Diese Fällung kann auch in Gegenwart von Ascorbinsäure durchgeführt werden.

Die gallertartigen Eisen(II)-alginat-Perlen werden unter
$N_2$-Schutzgas bei 4°C 12 Stunden lang in der Fällungslösung gehärtet, mit entgastem $H_2O$ gewaschen und im
Vakuum getrocknet.

Der Eisen(II)-gehalt beträgt 198 mg Eisen/g Trockenmaterial.

B e i s p i e l   2

Darstellung von Magnesiumalginat

10 g Alginsäure (Sigma) werden in 200 ml $H_2O$ suspendiert.
Unter Rühren wird eine Lösung von 5 g $Mg(OH)_2$ in 50 ml
$H_2O$ zugetropft, bis ein pH-Wert von 7 erreicht ist. Die
klare Lösung wird entweder am Rotationsverdampfer getrocknet oder gefriergetrocknet. Gefriergetrocknetes
Material besitzt eine schaumige (bimssteinähnliche)
Struktur, zeigt Gelbfärbung und ist gut wasserlöslich.
Der Magnesiumgehalt beträgt 85 mg/g Trockenmaterial.

B e i s p i e l    3

8 g Natriumalginat (Protanal LFR 5/60) werden in 100 ml
$H_2O$ gelöst und in 300 ml einer 0,5 M $ZnCl_2$-Lösung getropft, die mit NaOH auf pH 7 gebracht wurde. Die Zn-
Alginat-Perlen härten 12 Stunden bei 4°C in der Fällungslösung. Nach dem Auswaschen nichtgebundener Ionen (3 x
30 Sekunden in $H_2O$ suspendieren) werden die Zn-Alginat-
Perlen bei 40°C getrocknet.

Der Zinkgehalt beträgt 180 mg Zink/g Trockengewicht.

B e i s p i e l    4

<u>Darstellung eines Eisen-Magnesium-Alginatgels</u>

8 g Natriumalginat (Protanal) werden unter Rühren in
80 ml bidestilliertem Wasser gelöst, die Lösung mit 1 M
NaOH auf pH 7,4 gebracht und mit bidestilliertem Wasser
auf 100 ml aufgefüllt.

Diese 8%ige Na-Alginatlösung wird zur Härtung in 500 ml
einer Lösung, die 0,25 M an $FeCl_2$ und 0,4 M an $MgCl_2$ ist,
getropft. Beim Eintropfen bilden sich Perlen von Eisen-
Magnesium-Alginat, die bei einer Temperatur von 4°C
innerhalb von 12 Stunden vollständig durchhärten.

Zur Entfernung nichtstöchiometrisch gebundener Eisen-
und Magnesiumionen aus den Perlen wird mit Wasser gewaschen. Die Trocknung benötigt bei 40°C ca. 24 Stunden.

Der Gehalt beträgt 80 mg Eisen und 55 mg Magnesium pro
g Trockengewicht.

P A T E N T A N S P R U C H

Verwendung von Eisen(II)-, Zink- und Magnesiumsalzen
und ihren Mischsalzen

(1) der Alginsäure,

(2) der Pektinsäure,

(3) einer natürlichen polymeren, anionischen Carbonsäure aus der Gruppe Polymannuronsäure, Polygalacturonsäure, Polyglucuronsäure, Polyguluronsäure und
Polyiduronsäure oder ihren Gemischen

zur Substitution von Eisen, Zink und Magnesium.